# EUROPÄISCHE PATENTSCHRIFT

(11) **EP 4 439 070 B1**
(45) Veröffentlichungstag und Bekanntmachung des Hinweises auf die Patenterteilung: **03.12.2025**
(21) Anmeldenummer: 23164272.9
(22) Anmeldetag: 27.03.2023
(51) Int. Cl.: G01N 35/00

(54) **AUTOMATISCHES ANALYSESYSTEM**
AUTOMATIC ANALYSIS SYSTEM
SYSTÈME D'ANALYSE AUTOMATIQUE

(43) Veröffentlichungstag der Anmeldung: 02.10.2024
(73) Patentinhaber: Siemens Healthcare Diagnostics Products GmbH, 35041 Marburg (DE)
(72) Erfinder: Patzke, Juergen, 35043 Marburg (DE)
(74) Vertreter: Siemens Healthineers Patent Attorneys

(56) Entgegenhaltungen:
- WO-A1-2020/190266
- US-A1- 2009 035 346
- US-A1- 2012 060 541
- US-A1- 2012 329 082
- FLIEDER T. ET AL: "The Sysmex CS-5100 coagulation analyzer offers comparable analytical performance and excellent throughput capabilities", PRACTICAL LABORATORY MEDICINE, vol. 6, 1 December 2016 (2016-12-01), pages 38 - 47, XP093050374, ISSN: 2352-5517, DOI: 10.1016/j.plabm.2016.09.002
- M�LLER-BERGHAUS GERT ET AL: "Labor & Diagnose 16 H�mostase system", 1 January 2012 (2012-01-01), pages 1 - 233, XP093078582, Retrieved from the Internet <URL:https://www.labor-und-diagnose.de/k16.html> [retrieved on 20230904]
- PELZER H ET AL: "DETERMINATION OF HUMAN THROMBIN-ANTITHROMBIN III COMPLEX IN PLASMA WITH AN ENZYME-LINKED IMMUNOSORBENT ASSAY", THROMBOSIS AND HAEMOSTASIS, SCHATTAUER GMBH, DE, vol. 59, no. 1, 1 January 1988 (1988-01-01), pages 101 - 106, XP009021770, ISSN: 0340-6245
- KATO SEIJI ET AL: "Novel monoclonal antibody-based enzyme immunoassay for determining plasma levels of ADAMTS13 activity", TRANSFUSION, AMERICAN ASSOCIATION OF BLOOD BANKS, BETHESDA, MD, US, vol. 46, no. 8, 1 August 2006 (2006-08-01), pages 1444 - 1452, XP002499925, ISSN: 0041-1132, DOI: 10.1111/J.1537-2995.2006.00914.X
- N. A. ALSHAIKH ET AL: "New functional assays to selectively quantify the activated protein C- and tissue factor pathway inhibitor-cofactor activities of protein S in plasma", JOURNAL OF THROMBOSIS AND HAEMOSTASIS, vol. 15, no. 5, 20 March 2017 (2017-03-20), GB, pages 950 - 960, XP055647588, ISSN: 1538-7933, DOI: 10.1111/jth.13657
- LANGE U ET AL: "Ecarin chromogenic assay - A new method for quantitative determination of direct thrombin inhibitors like hirudin", PATHOPHYSIOLOGY OF HAEMOST THROMBOSIS, KARGER, CH, vol. 33, no. 4, 1 January 2003 (2003-01-01), pages 184 - 191, XP002385856, ISSN: 1424-8832
- LETERTRE L.R. ET AL: "A single test to assay warfarin, dabigatran, rivaroxaban, apixaban, unfractionated heparin, and enoxaparin in plasma", vol. 14, no. 5, 1 May 2016 (2016-05-01), pages 1043 - 1053, XP093078628, ISSN: 1538-7836, Retrieved from the Internet <URL:https://api.elsevier.com/content/article/PII:S1538783622035127?httpAccept=text/plain> DOI: 10.1111/jth.13300
- ANONYMOUS: "ASSERACHROM VWF:FVIIIB, Immunenzymatischer Nachweis der Faktor VIII-Bindungsf�higkeit des von Willebrand-Faktors", 1 February 2018 (2018-02-01), Asni�res sur Seine (France), pages 1 - 1, XP093078729, Retrieved from the Internet <URL:https://ifu.stago.com/fileadmin/user_upload/notices/Notices_Reactifs/0091904201802/DE_ASSERACHROM%23VWF%23FVIIIB_20180228.pdf> [retrieved on 20230905]

## Beschreibung

Die Erfindung betrifft ein automatisches Analysesystem zum Analysieren einer Probe mit einer Vorrichtung zur Lagerung von Kartuschen bei einer Temperatur, die weniger als 268,15 Kelvin beträgt.

Zahlreiche Nachweis- und Analyseverfahren zur Bestimmung physiologischer Parameter in Körperflüssigkeitsproben oder anderen biologischen Proben werden heute automatisiert in großer Anzahl in automatischen Analysegeräten, auch so genannten in vitro-Diagnostiksystemen, durchgeführt.

Heutige Analysegeräte sind in der Lage, eine Vielzahl von Nachweisreaktionen und Analysen mit einer Probe durchzuführen. Um eine Vielzahl von Untersuchungen automatisiert durchführen zu können, sind diverse Vorrichtungen zum räumlichen Transfer von Messzellen, Reaktionsbehältern und Reagenzbehältern erforderlich, wie z.B. Transferarme mit Greiffunktion, Transportbänder oder drehbare Transporträder, sowie Vorrichtungen zum Transfer von Flüssigkeiten, wie z.B. Pipettiervorrichtungen. Die Geräte umfassen eine Steuereinheit, die mittels entsprechender Software dazu in der Lage ist, die Arbeitsschritte für die gewünschten Analysen weitgehend selbstständig zu planen und abzuarbeiten.

Viele der in derartigen automatisiert arbeitenden Analysegeräten verwendeten Analyseverfahren beruhen auf optischen Methoden. Diese Verfahren ermöglichen den qualitativen und quantitativen Nachweis von Analyten, d.h. der nachzuweisenden oder zu bestimmenden Substanzen in Proben. Die Bestimmung klinisch relevanter Parameter, wie zum Beispiel der Konzentration oder Aktivität eines Analyten, erfolgt vielfach, indem ein Teil einer Probe mit einem oder mehreren Testreagenzien in einem Reaktionsgefäß, welches auch die Messzelle sein kann, vermischt wird, wodurch z.B. eine biochemische Reaktion oder eine spezifische Bindungsreaktion in Gang gesetzt wird, die eine messbare Veränderung einer optischen oder anderen physikalischen Eigenschaft des Testansatzes bewirkt.

In medizintechnischen Geräten wie z.B. diagnostischen Analysegeräten zur automatischen Analyse und Untersuchung von z.B. in-vitro Proben von biologischen Körperflüssigkeiten werden beispielsweise die benötigten Reagenzien mittels einer Pipettiervorrichtung mit einer Pipettiernadel in eine Messküvette eingefüllt. Die Messküvette wird dabei mit einem Küvettengreifer innerhalb des automatischen Analysegerätes zu verschiedenen Positionen mittels eines Robotorarms automatisch verfahren, der Teil einer Robotorstation ist. Nach der Messung wird die benutzte Messküvette zur Entsorgung durch einen Abfallschacht in einen Abfallbehälter verbracht.

In solchen automatischen Analysatoren werden Verbrauchsmaterialien, wie z.B. Reagenzien, Kontrollen und Standards im Gerät gespeichert, um einen automatischen Betrieb über eine längere Zeit bzw. zur Analyse von einer größeren Anzahl von Proben zu ermöglichen.

Spezielle Teste, die typischerweise nur selten in einem Labor angefordert werden, für bestehende, vollautomatische Analysensysteme zu entwickeln, ist im Allgemeinen vergleichsweise kostenaufwändig und daher häufig unrentabel.

Bereits etablierte, im Markt befindliche Teste können in der Regel nur mit zahlreichen und aufwändigen technischen Änderungen an die neuen Analysensysteme angepasst werden. Daher entstehen diesbezüglich meist hohe Entwicklungskosten. Die aktuell üblichen, vollautomatischen Analysensysteme sind auf Teste ausgelegt, die häufig abgearbeitet werden, so dass es sich lohnt, Reagenzien, Kontrollen und Standards in z.B. Kartuschen oder Flaschen auf dem Gerät vorzuhalten.

Bei selten durchgeführten Testen wird die Stabilitätsdauer dieser Komponenten häufig überschritten, so dass sie regelmäßig zu verwerfen sind, was zusätzliche Kosten verursacht und eine automatisierte Durchführung der Teste weiter unwirtschaftlich macht. Weiter sind für solche Teste vergleichsweise häufig Kalibrationen durchzuführen, teilweise für jede einzelne Probenmessung, was technisch aufwändig, zeitintensiv und wirtschaftlich unvorteilhaft ist.

Bisher gibt es im Wesentlichen einerseits vollautomatische Analysesysteme, die für häufig durchzuführende Teste technisch optimiert sind, und andererseits entweder Point-of-Care-Teste oder ELISAs (Enzyme-linked Immunosorbent Assays) oder andere Lösungen mit diversen manuellen Schritten, jedoch keine vollautomatisierte Lösung für sehr seltene, spezielle Teste.

US 2012/0329082 A1 offenbart bekannte Vorrichtungen, Systeme und Verfahren zur Hämostasebewertung.

Die Vorrichtungen aus dem Stand der Technik ermöglichen also gewöhnlich nicht eine einfache und rentable Entwicklung und Bereitstellung von nur selten in einem Labor angeforderten Testen auf vollautomatischen Analysensystemen.

Es ist daher Aufgabe der Erfindung, eine vereinfachte Entwicklung sowie Bereitstellung von Testen zur Durchführung auf vollautomatisierten Analysesystemen zu ermöglichen, insbesondere auch für nur selten in einem Labor angeforderten Teste.

**Diese Aufgabe wird erfindungsgemäß durch die im Folgenden beschriebenen Gegenstände und Verfahren gelöst.**

Es wurde gefunden, dass eine verbesserte, vereinfachte Entwicklung sowie Bereitstellung von Testen zur Durchführung von nur selten in einem Labor angeforderten Teste auf vollautomatisierten Analysesystemen erreicht werden kann, durch eine Verwendung von eingefrorenen Reagenzien, die in einer Kartusche zusammen mit Messküvetten vorgesehen werden.

Dabei werden an den jeweiligen Test angepasste Kartuschen verwendet sowie eingefrorene Reagenzien und/oder Kontrollen sowie Messküvetten, wobei sich alle diese Komponenten in der Kartusche befinden.

Dies hat den Vorteil, dass der Entwicklungsaufwand insbesondere für nur selten in einem Labor angeforderte Teste erheblich reduziert werden kann und gleichzeitig solche Teste optimiert auf vollautomatisierten Analysesystemen bereitgestellt und in Laboren vorgehalten und durchgeführt werden können.

Gegenstand der vorliegenden Erfindung ist insbesondere ein automatisches Analysesystem zum Analysieren einer Probe, das Analysesystem umfassend eine erste Vorrichtung zur Lagerung von Kartuschen bei einer ersten Temperatur, wobei die erste Temperatur weniger als 268,15 Kelvin beträgt, bevorzugt eine zweite Vorrichtung zur Aufnahme einer Transportbox, eine dritte Vorrichtung zur Lagerung von Kartuschen bei einer zweiten Temperatur, wobei die zweite Temperatur mehr als 273,15 Kelvin beträgt, eine vierte Vorrichtung zum Erwärmen der Kartuschen von der ersten Temperatur auf die zweite Temperatur, eine fünfte Vorrichtung zum Pipettieren mindestens einer Flüssigkeit, eine sechste Vorrichtung zum Inkubieren mindestens einer Kartusche, eine siebte Vorrichtung zum Messen mindestens eines Probenansatzes der Probe, wobei die Kartuschen jeweils mindestens ein erstes Kompartiment mit mindestens einem Reagenz, mindestens ein zweites Kompartiment mit mindestens einer Kontrolle, und mindestens ein drittes Kompartiment umfassen, wobei das dritte Kompartiment eine Messküvette umfasst, wobei eine Messung des Testansatzes mittels der siebten Vorrichtung in der Messküvette erfolgen kann, wobei dem Analysesystem mindestens eine Kartusche zugeführt werden kann, wobei die Kartusche eine dritte Temperatur aufweist, wobei die dritte Temperatur weniger als 268,15 Kelvin beträgt, wobei bevorzugt die Transportbox zum Transport mindestens einer Kartusche bei der dritten Temperatur ausgestaltet ist.

Bevorzugt wird die Kartusche dem Analysesystem in der Transportbox zugeführt. Die Kartusche befindet sich also bevorzugt bei Zuführung in das Analysesystem hinein wenigstens teilweise, bevorzugt vollständig, innerhalb der Transportbox. Bevorzugt wird die Transportbox mit einer oder mehreren Kartuschen darin dem Analysesystem entsprechend zugeführt.

Die dritte Vorrichtung ist bevorzugt als ein Block ausgestaltet, der bevorzugt insgesamt bei 37 Grad Celsius temperiert ist. Bevorzugt ist in diesem Block eine separate Einrichtung enthalten, z.B. ein Wasserbad und/oder ein Metallblock, die für das Auftauen der Kartuschen verwendet wird, und die bevorzugt die vierte Vorrichtung umfasst oder diese darstellt.

Weiter umfasst die dritte Vorrichtung bevorzugt einen Inkubationsblock für den Probenansatz, wobei die Inkubation z.B. an der Luft, in einem Wasserbad und/oder einem Metallblock erfolgten kann, der die sechste Vorrichtung umfasst oder diese darstellt.

Weiter umfasst die dritte Vorrichtung bevorzugt eine Messeinheit, bei der die Messung der Probe bevorzugt in einem Luftraum erfolgt, also sich die Probe bei der Messung bevorzugt nicht in einem Wasserbad und/oder einem Metallblock befindet. Bevorzugt umfasst die siebte Vorrichtung diese Messeinheit oder stellt diese dar.

Bevorzugt sind die erste Temperatur und die dritte Temperatur gleich, sie können jedoch auch unterschiedlich sein.

Letzteres ist besonders dann vorteilhaft, wenn der Rücktransport und/oder Weitertransport der Kartuschen bei Raumtemperatur erfolgt.

In einer bevorzugten Ausführung ist die Transportbox weiter zum Transport mindestens einer Kartusche bei einer vierten Temperatur ausgestaltet, wobei die vierte Temperatur 233,15 bis 323,15 Kelvin, bevorzugt 288,15 bis 298,15 Kelvin, besonders bevorzugt Raumtemperatur beträgt.

In einer weiteren bevorzugten Ausführung umfasst die dritte Vorrichtung die vierte Vorrichtung, die fünfte Vorrichtung, die sechste Vorrichtung und/oder die siebte Vorrichtung wenigstens teilweise, bevorzugt vollständig. Also umfasst die dritte Vorrichtung vorteilshafterweise verschiedene andere Vorrichtungen und integriert diese in eine Vorrichtung.

In einer weiteren bevorzugten Ausführung ist die zweite Temperatur eine vorbestimmte, konstante Temperatur zwischen 293,15 und 315,15 Kelvin, bevorzugt zwischen 308,15 und 312,15 Kelvin ist, wobei besonders bevorzugt die zweite Temperatur 310,15 Kelvin beträgt.

In einer weiteren bevorzugten Ausführung beträgt die erste Temperatur weniger als 268,15 Kelvin, bevorzugt weniger als 263,15 Kelvin, besonders bevorzugt 255,15 bis 245,15 Kelvin.

In einer weiteren bevorzugten Ausführung umfasst die Flüssigkeit eine Probenflüssigkeit.

In einer weiteren bevorzugten Ausführung umfasst die fünfte Vorrichtung eine Pipettiernadel zum Pipettieren von Proben, Reagenzien, Waschlösungen und/oder eines Verdünnungsmediums, wobei das Verdünnungsmedium bevorzugt eine Pufferlösung umfasst.

In einer weiteren bevorzugten Ausführung umfasst die zweite Vorrichtung einen Schacht, wobei der Schacht zur Aufnahme mindestens einer Transportbox ausgestaltet ist.

In einer weiteren bevorzugten Ausführung umfasst das Analysesystem eine achte Vorrichtung zur Steuerung der ersten, zweiten, dritten und/oder vierten Temperatur.

In einer weiteren bevorzugten Ausführung umfasst das Analysesystem eine neunte Vorrichtung zur automatischen Verbringung mindestens einer Kartusche aus einer sich in der zweiten Vorrichtung befindenden Transportbox heraus und in die erste Vorrichtung hinein.

In einer weiteren bevorzugten Ausführung umfasst das Analysesystem eine zehnte Vorrichtung zur automatischen Verbringung mindestens einer Kartusche aus der ersten Vorrichtung heraus und in die dritte Vorrichtung hinein.

In einer weiteren bevorzugten Ausführung umfasst das Analysesystem eine elfte Vorrichtung zur automatischen Verbringung mindestens einer Kartusche aus der dritten Vorrichtung heraus in eine sich in der zweiten Vorrichtung befindenden Transportbox hinein.

Ein weiterer bevorzugter Gegenstand der Erfindung ist insbesondere eine Transportbox für ein erfindungsgemäßes automatisches Analysesystem zum Transport mindestens einer Kartusche zu dem Analysesystem hin und/oder von dem Analysesystem weg, wobei die Kartusche mindestens ein erstes Kompartiment mit mindestens einem gefrorenen Reagenz, mindestens ein zweites Kompartiment mit mindestens einer Kontrolle, und mindestens ein drittes Kompartiment umfasst, wobei das dritte Kompartiment eine Messküvette umfasst, wobei bevorzugt sich in der Transportbox mindestens eine Kartusche befindet.

Ein weiterer Gegenstand der Erfindung ist insbesondere eine, bevorzugt mittels einer erfindungsgemäßen Transportbox, zu einem erfindungsgemäßen automatischen Analysesystem hin und/oder von dem Analysesystem weg transportierbare Kartusche, wobei die Kartusche mindestens ein erstes Kompartiment mit mindestens einem gefrorenen Reagenz, mindestens ein zweites Kompartiment mit mindestens einer Kontrolle, und mindestens ein drittes Kompartiment umfasst, wobei das dritte Kompartiment eine Messküvette umfasst.

Ein weiterer Gegenstand der Erfindung ist insbesondere eine Verwendung einer erfindungsgemäßen Transportbox und/oder einer erfindungsgemäßen Kartusche in einem erfindungsgemäßen automatischen Analysegerät.

Ein weiterer Gegenstand der Erfindung ist insbesondere ein Verfahren zum Analysieren einer Probe mittels eines erfindungsgemäßen automatischen Analysesystems unter Verwendung einer erfindungsgemäßen Kartusche und bevorzugt einer erfindungsgemäßen Transportbox, das Verfahren umfassend die folgenden Schritte:
- Zuführung der Kartusche, bevorzugt in der Transportbox, zu dem automatischen Analysesystem, wobei die Kartusche die erste Temperatur aufweist,
- bevorzugt Verbringung der Transportbox mit der Kartusche in die zweite Vorrichtung,
- bevorzugt Entnahme der Kartusche aus der Transportbox heraus und Zuführung in die erste Vorrichtung mittels der neunten Vorrichtung, und/oder Zuführung der Kartusche (5) in die erste Vorrichtung (2),
- Entnahme der Kartusche aus der ersten Vorrichtung und Zuführung in die dritte Vorrichtung hinein mittels der zehnten Vorrichtung,
- Erwärmung der Kartusche von der ersten Temperatur auf die zweite Temperatur mittels der vierten Vorrichtung,
- Pipettierung mindestens einer Probe, mindestens eines Reagenz und mindestens einer Kontrolle mittels der fünften Vorrichtung umfassend die Herstellung eines Testansatzes,
- bevorzugt Inkubation des Testansatzes in der Kartusche mittels der sechsten Vorrichtung,
- Messung des Testansatzes mittels der siebten Vorrichtung in der Messküvette des dritten Kompartiments der Kartusche,
- Auswertung der Messung des Testansatzes unter Verwendung mindestens einer Kalibrationskurve, die bevorzugt elektronisch an das Analysesystem übermittelten wurde,
- bevorzugt Verbringung der Kartusche aus der dritten Vorrichtung hinaus in die Transportbox hinein mittels der elften Vorrichtung,
- bevorzugt Transport der Kartusche in der Transportbox zu einer Entsorgungsstation, wobei die Kartusche die vierte Temperatur aufweist.

Bevorzugt erfolgt die Zuführung der Kartusche (5) in die erste Vorrichtung (2) dabei manuell und/oder unmittelbar und bevorzugt auf direktem Weg, ohne dass die Kartusche (5) zuvor in eine andere Vorrichtung des Analysesystems zugeführt wird.

In einer bevorzugten Ausführung umfasst das Verfahren die Durchführung eines Hämostase-Testes, bevorzugt eines Faktor VIII-Bindungstests.

In weiteren bevorzugten Ausführungen umfasst das Verfahren die Durchführung eines oder mehrerer der Teste und/oder Gruppen von Testen, die beschrieben sind in Kapitel 16 "Hämostasesystem" unter den Punkten 16.10 bis 16.28 in "Labor und Diagnose: Indikation und Bewertung von Laborbefunden für die medizinische Diagnostik" von Lothar Thomas, 8. Auflage 2012, ISBN-10: 3980521583, ISBN-13: 978-3980521581, und/oder in den Kapiteln "Laboratory investigations" in der elektronisch im Internet unter https://www.clinical-laboratory-diagnostics.com verfügbaren Publikation "Clinical Laboratory Diagnostics" von Lothar Thomas, released 15.12.2022.

In weiteren bevorzugten Ausführungen umfasst das Verfahren die Durchführung eines oder mehrerer der im Folgenden angegebenen Teste und/oder Gruppen von Testen.

ELISA-Test zur Bestimmung der Konzentration des Thrombin-Antithrombin-Komplexes als Marker für prothrombotische Zustände (Pelzer, Hermann, Angela Schwarz, and Norbert Heimburger. "Determination of human thrombin-antithrombin III complex in plasma with an enzyme-linked immunosorbent assay." Thrombosis and haemostasis 59.01 (1988): 101-106.)

ELISA-Test zur Bestimmung der ADAMTS13-Aktivität mit anti-N10 monoklonalem Antikörper zur Erkennung einer thrombotischenthrombozytopenischen (Purpura Kato, Seiji, et al. "Novel monoclonal antibody-based enzyme immunoassay for determining plasma levels of ADAMTS13 activity." Transfusion 46.8 (2006): 1444-1452.)

Test zur Bestimmung der Protein S-TFPI-Kofaktor Aktivität mittels Messung der Thrombingenerierung (Alshaikh, N. A., et al. "New functional assays to selectively quantify the activated protein C-and tissue factor pathway inhibitorcofactor activities of protein S in plasma." Journal of Thrombosis and Haemostasis 15.5 (2017): 950-960.)

Test Ecarin Chromogenic Assay zur Quantifizierung der antikoagulatorischen Wirkung von direkten Thrombin-Hemmsubstanzen (Lange, U., G. Nowak, and E. Bucha. "Ecarin chromogenic assay-a new method for quantitative determination of direct thrombin inhibitors like hirudin." Pathophysiology of haemostasis and thrombosis 33.4 (2003): 184-191.)

Test dFiix-PT zur Ermittlung des antikoagulatorischen Effekts von Warfarin, Dabigatran, Rivaroxaban und Apixaban (Letertre, L. R., et al. "A single test to assay warfarin, dabigatran, rivaroxaban, apixaban, unfractionated heparin, and enoxaparin in plasma." Journal of Thrombosis and Haemostasis 14.5 (2016): 1043-1053.).

In bevorzugten Ausführungsformen erfolgt keine Inkubation des Testansatzes, oder es erfolgen beliebig viele Inkubationsschritte, z.B. eins bis zehn Inkubationsschritte. Darunter können insbesondere auch Inkubationsschritte von Reagenzmischungen sein, die noch keine Probe enthalten, d.h. der Testansatz besteht bei einem solchen Inkubationsschritt aus einem Reagenz und/oder einer Reagenzmischung und umfasst noch keine Probe.

Teste können erfindungsgemäß Kalibrationen bedürfen. Bevorzugt erfolgen dabei Kalibrationen über Einpunkt- und/oder Zeitpunktkalibrationen, was bedeutet, dass eine Kalibrationskurvenform elektronisch zur Verfügung steht und die Lage der Kalibrationskurve durch die Messung eines einzigen oder von höchsten zwei Messpunkten festgelegt wird. Bevorzugt umfassen die Kartuschen einen oder mehrere Kalibratoren für die Durchführung einer oder mehrerer Kalibrationen. Die Kalibratoren sind dabei bevorzugt in einem oder mehreren Kompartimenten der Kartusche angeordnet, die bevorzugt spezifisch hierfür ausgestaltet sind.

In einer weiteren bevorzugten Ausführungsform umfasst das Verfahren weiter die folgenden Schiritte:
- automatische Nachbestellung von verbrauchten Kartuschen.

In einer weiteren bevorzugten Ausführungsform umfasst das Verfahren weiter die folgenden Schiritte:
- automatische Ermittlung, welche Teste und/oder wie viele Kartuschen in dem Analysesystem vorzuhalten sind, und bevorzugt entsprechende automatische Bestellung und/oder Nachbestellung von Testen und/oder Kartuschen.

Unter einer "Probe" ist im Sinne der Erfindung das Material zu verstehen, dass die nachzuweisende Substanz (den Analyten) vermutlich enthält. Der Begriff "Probe" umfasst insbesondere biologische Flüssigkeiten von Menschen oder Tieren wie z.B. Blut, Plasma, Serum, Sputum, Exsudat, bronchoalveoläre Lavage, Lymphflüssigkeit, Synovialflüssigkeit, Samenflüssigkeit, Vaginalschleim, Feces, Urin, Liquor, aber auch z.B. durch Homogenisation oder Zelllyse für die photometrische, bevorzugt nephelometrische Bestimmung entsprechend aufgearbeitete Gewebe- oder Zellkulturproben. Ferner können auch z.B. pflanzliche Flüssigkeiten oder Gewebe, forensische Proben, Wasser- und Abwasserproben, Nahrungsmittel, Arzneimittel als Probe dienen, die ggf. vor der Bestimmung einer entsprechenden Probenvorbehandlung zu unterziehen sind.

Unter einem "Reagenz" ist im Sinne der Erfindung ein Material zu verstehen, das Stoffe enthält, die bei Kontakt mit anderen Stoffen aus Reagenzien und Stoffen aus der Probe spezifische Reaktionen hervorrufen, welche physikalisch messbar sind und genutzt werden können, um einen Analyten aus einer Probe zu messen. Insbesondere umfassen Reagenzien auch Puffer, wässrigen NaCl-Lösungen und/oder einfach- oder mehrfach destilliertes Wasser.

Unter einem "Puffer" ist im Sinne der Erfindung ein Material zu verstehen, das allein, oder unter anderem dazu dient, den PH-Wert im Reaktionsansatz konstant zu halten. Insbesondere umfassen Puffer auch wässrigen NaCl-Lösungen und/oder einfach- oder mehrfach destilliertes Wasser.

Bei einem quantitativen Nachweis wird die Menge, die Konzentration oder die Aktivität des Analyten in der Probe gemessen. Dabei umfasst der Begriff Analyt auch Parameter, die die Funktionswege von Stoffwechselvorgängen erfassen wie zum Beispiel die aktivierte partielle Thromboplastinzeit (APTT). Von dem Begriff des "quantitativen Nachweises" sind auch semiquantitative Methoden umfasst, die nur die ungefähre Menge, Konzentration oder Aktivität des Analyten in der Probe erfassen oder nur zu einer relativen Mengen-, Konzentrations- oder Aktivitätsangabe dienen können. Unter einem qualitativen Nachweis ist der Nachweis des Vorhandenseins des Analyten in der Probe überhaupt oder das Anzeigen, dass die Menge, Konzentration oder Aktivität des Analyten in der Probe unterhalb oder oberhalb eines bestimmten oder mehrerer bestimmter Schwellenwerte liegt, zu verstehen.

Bei einer Messküvette handelt es sich beispielsweise um eine Küvette oder ein Reaktionsgefäß aus Glas, Kunststoff oder Metall. Vorteilhafterweise ist die Messküvette aus optisch transparenten Materialien gefertigt, was besonders beim Einsatz von optischen Analyseverfahren vorteilhaft sein kann.

Die Begriffe "Messküvette" und "Küvette" werden synonym verwendet und bezeichnen den gleichen Gegenstand.

**Die Erfindung wird anhand von Zeichnungen exemplarisch näher erläutert. Darin zeigen:**
FIG 1 schematisch den Aufbau eines automatischen Analysesystems (1), eine Kartusche (3), eine Transportbox (5) und ein Zentrallager (25); FIG 2 schematisch Details einer Kartusche (3) transportierbar mittels der Transportbox (5);
FIG 3 schematisch ein Verfahren (30) zum Analysieren einer Probe (23) mittels eines automatischen Analysesystems (1).

**Gleiche Teile sind in allen Figuren mit denselben Bezugszeichen versehen.**

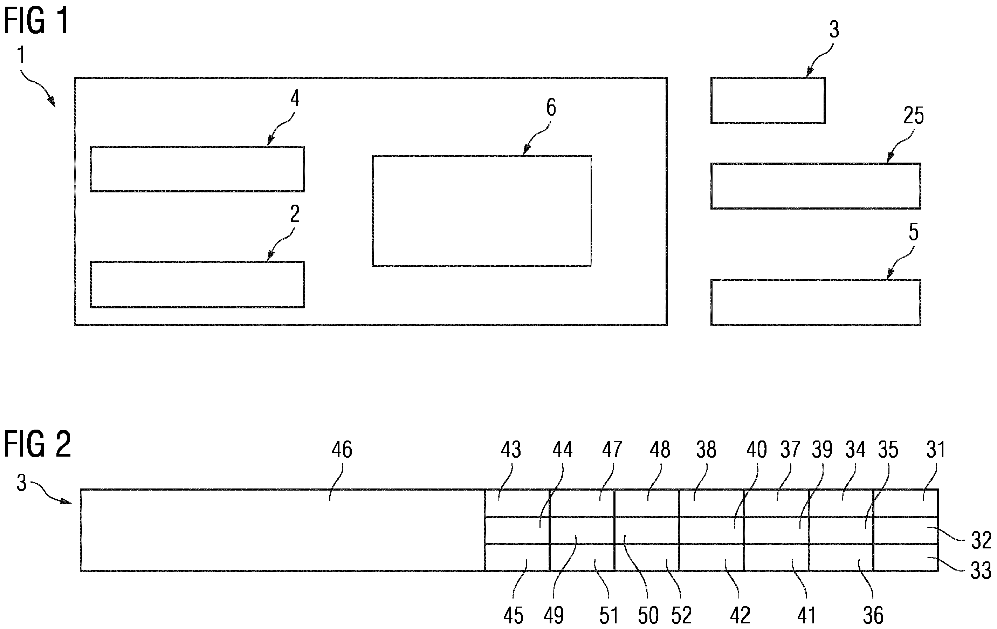

FIG 1 zeigt ein automatisches Analysesystems (1), eine Kartusche (3), eine Transportbox (5) und ein Zentrallager (25). Das automatische Analysesystems (1) ist zum automatischen Analysieren einer Probe (23) ausgestaltet. Das Analysesystem (1) umfasst eine erste Vorrichtung (2) zur Lagerung von Kartuschen (3) bei einer ersten Temperatur (T1). Die erste Temperatur (T1) beträgt dabei weniger als 268,15

Kelvin. Weiter umfasst das automatische Analysesystems (1) eine zweite Vorrichtung (4) zur Aufnahme einer Transportbox (5) sowie eine dritte Vorrichtung (6) zur Lagerung, bzw. erfindungsgemäßen Verwendung, von Kartuschen (3) bei einer zweiten Temperatur (T2). Die zweite Temperatur (T2) beträgt dabei mehr als 273,15 Kelvin. Die dritte Vorrichtung (6) umfasst eine vierte Vorrichtung (7) zum Erwärmen der Kartuschen (3) von der ersten Temperatur (T1) auf die zweite Temperatur (T2), eine fünfte Vorrichtung (8) zum Pipettieren mindestens einer Flüssigkeit, eine sechste Vorrichtung (9) zum Inkubieren mindestens einer Kartusche (3) und eine siebte Vorrichtung (10) zum Messen mindestens eines Probenansatzes (11) der Probe (23). Die vierte Vorrichtung (7), fünfte Vorrichtung (8), sechste Vorrichtung (9) und siebte Vorrichtung (10), die Probe (23) sowie der Probenansatz (11) sind nicht dargestellt.

FIG 1 zeigt weiter schematisch eine erfindungsgemäße Kartusche (3) transportierbar mittels einer erfindungsgemäßen Transportbox (5). Die Kartusche (3) umfasst jeweils ein erstes Kompartiment (12) mit einem gefrorenen Reagenz (13), ein zweites Kompartiment (14) mit einer Kontrolle (15), und ein drittes Kompartiment (16). Das dritte Kompartiment (16) umfasst eine Messküvette (17). Eine Messung des Testansatzes (11) erfolgt erfindungsgemäß mittels der siebten Vorrichtung (10) in der Messküvette (17). Die Kompartimente (12, 14, 16), das Reagenz (13), die Kontrolle (15) sowie die Messküvette (17) sind nicht dargestellt.

FIG 1 zeigt weiter schematisch eine erfindungsgemäße Transportbox (5). Die Transportbox (5) ist zum Transport mindestens einer Kartusche (3) bei einer dritten Temperatur (T3) ausgestaltet. Die dritte Temperatur (T3) beträgt weniger als 268,15 Kelvin.

FIG 1 zeigt weiter schematisch ein erfindungsgemäßes Zentrallager (25).

FIG 2 zeigt schematisch Details einer erfindungsgemäßen Kartusche (3), die so ausgestaltet ist, dass sie mittels einer erfindungsgemäßen Transportbox (5) transportiert werden kann.

Die Kartusche (3) ist in Aufsicht gezeigt und es sind verschiedene Kompartimente schematisch dargestellt. Die Abmessungen der Kartusche (3) betragen in der Länge 100 mm, in der Breite 20 mm und in der Höhe 15 mm. Die Kartusche (3) umfasst 22 individuelle Küvetten (31 bis 52) 1 bis 22, von denen 21 Küvetten (31 bis 45 und 47 bis 52) gleichartig, insbesondere in Größe und Form, ausgestaltet sind. Diese 21 Küvetten (31 bis 45 und 47 bis 52) sind in drei Reihen zu je 7 Küvetten blockartig in Aufsicht rechts in der Kartusche (3) angeordnet und nehmen die Hälfte des Volumens der Kartusche (3) ein. Eine weitere Küvette (46) nimmt in Aufsicht linkes die andere Hälfte des Volumens der Kartusche (3) ein. Die Küvetten (31 bis 52) weisen jeweils oben eine Öffnung über im Wesentlichen den gesamten Querschnitt der jeweiligen Küvette auf.

Ein erfindungsgemäßes Anwendungsbeispiel für das automatische Analysesystem (1), die Transportbox (5) und die Kartusche (3) ist eine Durchführung eines Faktor VIII-Bindungstests, der als Enzyme-linked immunosorbent assay (ELISA) ausgestaltet ist. Der Faktor VIII-Bindungstest misst die Fähigkeit des von Willebrand Faktors, Faktor VIII zu binden und ist notwendig, um den Subtyp 2N des von Willebrand Syndroms zu diagnostizieren. Dieser Subtyp tritt sehr selten auf, aber eine Subtypendifferenzierung beim von Willebrand Syndrom ist sehr wichtig, um die richtigen Therapieentscheidungen zu treffen. Es gibt bisher keinen vollautomatischen Test sondern meist nur Tests im ELISA-Format. Diese sind vergleichsweise kostenaufwändig in der Anschaffung und Abarbeitung. Die Abarbeitung beinhaltet bisher teilweise manuelle Schritte, wodurch auch hohe Personalkosten entstehen können. Bei jedem Lauf ist üblicherweise eine Kalibrationskurve in Mehrfachbestimmung aufwändig neu zu erstellt. Auch kann die Präzision der Tests teilweise verbesserungswürdig sein.

Bei einem solchen Faktor VIII-Bindungstests kann es sich beispielsweise um eine Diagnosemethode bezüglich des Kits ASSERACHROM VWF:FVIIIB von Diagnostica Stago S.A.S. (Frankreich, Packungsbeilage Version Februar 2018, REF 00919, https://ifu.stago.com/fileadmin/user_upload/notices/Notices_R eactifs/0091904201802/DE_ASSERACHROM%23VWF%23FVIIIB_20180228. pdf abgerufen am 16.03.2023) handeln.

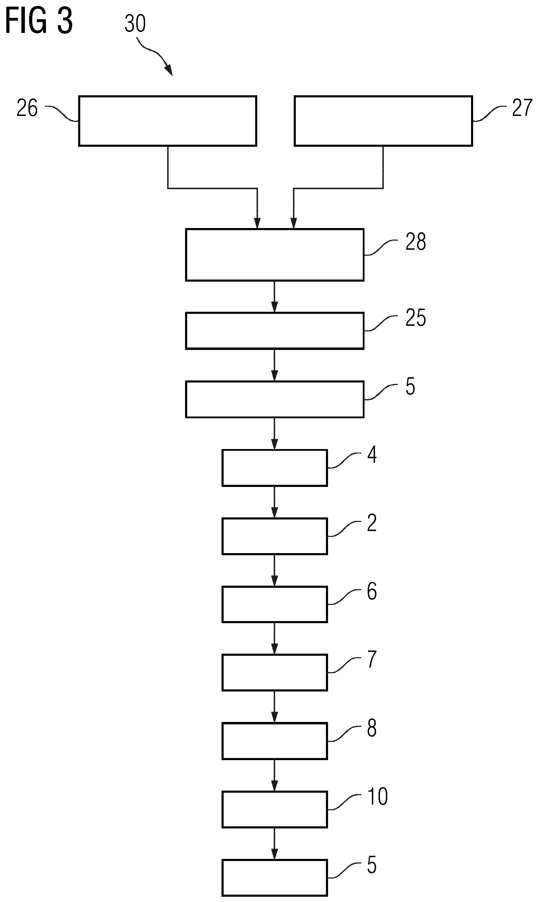

FIG 3 zeigt schematisch ein Verfahren (30) zum Analysieren einer Probe (23) mittels eines automatischen Analysesystems (1) hinsichtlich eines Faktor VIII-Bindungstests, der als Enzyme-linked immunosorbent assay (ELISA) ausgestaltet ist.

Zunächst werden bevorzugt testspezifische Kartuschen (3) mit Kompartimenten für Reagenzien und Kontrollen und mit Küvetten für die Messungen hergestellt (Herstellung Kartuschen (27)).

Weiter werden die benötigten Reagenzien eines bevorzugt sich auf dem Markt befindlichen, zugelassenen Tests bevorzugt unverändert hergestellt und in gebrauchsfertiger Form in die Kompartimente der Kartusche (3) gegeben (Herstellung Reagenzien (26)).

Dabei ergeben sich beispielsweise folgende Füllvolumina und Nutzungen für die einzelnen Küvetten (31 bis 52).

Die Küvette 16 (46) weist ein Füllvolumen von 8000 µL auf. Die Küvetten 1 bis 6 (31 bis 36) sind für die Durchführung von Messungen vorgesehen und weisen jeweils beschichtete Küvettenböden auf. Die Küvetten 7 bis 12 (37 bis 42) weisen ein Füllvolumen von 160 µL auf. Die Küvetten 17 bis 22 (47 bis 52) sind leer, d.h. weisen ein Füllvolumen von 0 µL auf.

Die Küvette 13 (43) weist ein Füllvolumen von 85 µL auf. Die Küvetten 14 und 15 (44 und 45) weisen ein Füllvolumen von 110 µL auf.

Dabei werden die Küvetten (31 bis 52) gemäß der oben angegebenen Füllvolumina jeweils mit folgenden Substanzen gefüllt, bzw. deren Boden entsprechend beschichtet.

Die Küvettenböden der Küvetten 1 bis 6 (31 bis 36) sind beschichtet mit F(ab')2 - Fragmenten Kaninchenantikörper gegen humanen VWF (von Willebrand Faktor).

Die Küvette 7 und 8 (37 und 38) sind gefüllt mit Reagenz mit rekombinanten humanen Faktor VIII.

Die Küvetten 9 und 10 (39 und 40) sind gefüllt mit Reagenz mit Peroxidase-gekoppeltem monoklonalen Maus-Antikörper gegen humanen Faktor VIII.

Die Küvetten 11 und 12 (41 und 42) sind gefüllt mit Reagenz mit Tetramethylbenzidin (TMB).

Die Küvette 13 (43) ist gefüllt mit Reagenz mit H2SO4.

Die Küvette 14 (44) umfasst die Kontrolle 1 und ist gefüllt mit Humanplasma, das eine bekannte Menge an VWF:FVIIIB im Normalbereich enthält.

Die Küvette 15 (45) umfasst die Kontrolle 2 und ist gefüllt mit Humanplasma, das eine bekannte Menge an VWF:FVIIIB im pathologisch niedrigen Bereich enthält.

Die Küvette 16 (46) ist mit Waschlösung, die zum Waschen der Messküvetten benutzt wird, gefüllt.

Die Küvetten 17 bis 22 (47 bis 52) sind leer.

Nach erfolgter Befüllung (28) werden die Kompartimente der Küvetten 1 bis 22 (31 bis 52) der Kartusche (3) mit einer Folie verschlossen.

Die Kartusche (3) wird eingefroren und in ein Zentrallager (25) überführt und dort bei weniger oder gleich minus 20 Grad Celsius aufbewahrt.

Anschließend wird die Kartusche (3), oder alternativ mehrere Kartuschen (3), in eine Transportbox (5) verfrachtet und bei weniger oder gleich minus 20 Grad Celsius zu einem Analysesystem (1) transportiert. Dabei löst eine Bestellung einer bestimmten Auswahl und Anzahl an Kartuschen (3) jeweils die entsprechenden Überführungen in die Transportbox (5) und den Transport zu dem Standort des Analysesystems (1) aus.

Am Standort des Analysesystems (1) wird die Transportbox (5) in die zweite Vorrichtung (4) eingeführt.

Das Analysesystem (1) entnimmt die Kartusche (3) automatisiert aus der Transportbox (5) und verbringt sie in die erste Vorrichtung (2) zur Lagerung, wobei die Temperatur der ersten Vorrichtung (2) weniger oder gleich minus 20 Grad Celsius beträgt.

Eine Platzierung einer Probe (23) auf dem Analysesystem (1) löst eine Überführung der für diese Probe (23) benötigen Kartusche (3) in die dritte Vorrichtung (6) aus.

In der dritten Vorrichtung (6) wird die Kartusche (3) mittels der vierten Vorrichtung (7) aufgetaut und bevorzugt mittels eines Wasserbades auf 37 Grad Celsius temperiert.

Anschließend erfolgt eine Überführung der bei 37 Grad Celsius temperierten Kartusche (3) in die fünfte Vorrichtung (8) und dann eine Abarbeitung des jeweiligen Tests einschließlich Pipettierschritten, Waschschritten und Inkubationsschritten.

Dabei erfolgen Inkubationsschritte bevorzugt in der sechsten Vorrichtung (9). Die verschiedenen Pipettierschritte, Waschschritte und Inkubationsschritte erfolgen dabei in der folgenden Abfolge.
i) Jeweils 50 µL Probe (23), 50 µL Kontrolle 1 und 50 µL Kontrolle 2 werden in zwei der Messküvetten pipettiert, wobei die Abdeckungsfolie durchstochen wird;
ii) Inkubation für 30 Minuten bei 37 Grad Celsius;
iii) Fünfmaliges Waschen mit jeweils 75 µL der Waschlösung
iv) Pipettierung von 50 µL Reagenz a. (Reagenz mit rekombinanten humanen Faktor VIII) in jede der 6 Messküvetten;
v) Inkubation für 30 Minuten bei 37 Grad Celsius;
vi) Fünfmaliges Waschen mit jeweils 75 µL der Waschlösung;
vii) Pipettierung von 50 µL Reagenz b. (Reagenz mit Peroxidase-gekoppeltem monoklonalen Maus-Antikörper gegen humanen Faktor VIII) in jede der 6 Messküvetten;
viii) Inkubation für 30 Minuten bei 37 Grad Celsius;
ix) Fünfmaliges Waschen mit jeweils 75 µL der Waschlösung;
x) Pipettierung von 50 µL Reagenz c. (Reagenz mit Tetramethylbenzidin (TMB)) in jede der 6 Messküvetten;
xi) Inkubation für 2,5 Minuten bei 37 Grad Celsius;
xii) Pipettierung von 12,5 µL Reagenz e. (Reagenz mit H2SO4 (1M) in jede der 6 Messküvetten;
xiii) Inkubation für 7,5 Minuten bei 37 Grad Celsius.

Bei der Probe (23) handelt es sich dabei um 0.11 M CitratPlasma, welches mit Phosphatpuffer auf einen VWF:Ag-Wert von 10% der Norm verdünnt worden war. Die VWF:Ag Konzentration der Probe muss zuvor bestimmt worden sein, damit der Verdünnungsfaktor festgelegt werden kann.

Anschließend erfolgt eine Überführung der Kartusche (3) in die siebte Vorrichtung (10) zur Messung des Probenansatzes und zur Auswertung der Messergebnisse. Dabei erfolgt eine Messung der Extinktion des Probenansatzes bei einer Wellenlänge von 450 nm. Die Auswertung erfolgt an einer elektronisch übermittelten Kalibrationskurve, welche unter Anwendung genau der entsprechenden Testprozedur zentral für die jeweilige Charge der Kartusche (3) ermittelt wurde.

Weiter wird die gebrauchte Kartusche (3) in die Transportbox (5) überführt. Die Transportbox ist jetzt nicht mehr gekühlt und weist vorteilshafterweise Raumtemperatur auf. Die Kartusche (3) verbleibt in der Transportbox (5) bis zum Austausch der Transportbox (5) gegen eine neu angelieferte, weitere Transportbox (5). Dabei ist die neu angelieferte Transportbox (5) wiederum mit gefrorenen Kartuschen (3) beladen. Die gebrauchte Kartusche (3) wird bevorzugt möglichst umweltschonend entsorgt oder einer Wiederverwertung zugeführt. Die Transportbox (5), in der die gebrauchte Kartusche (3) befördert wurde, wird gereinigt und für den nächsten Transport von weiteren, neuen Kartuschen (3) vorbereitet.

### Bezugszeichenliste

- 1: Analysesystem
- 2: Erste Vorrichtung (zur Lagerung von Kartuschen bei T1)
- 3: Kartusche
- T1: erste Temperatur
- 4: zweite Vorrichtung (zur Aufnahme einer Transportbox)
- 5: Transportbox
- 6: dritte Vorrichtung (zur Lagerung der Kartuschen bei T2)
- T2: zweite Temperatur
- 7: vierte Vorrichtung (zum Erwärmen der Kartuschen von T1 auf T2)
- 8: fünfte Vorrichtung (zum Pipettieren mind. einer Flüssigkeit)
- 9: sechste Vorrichtung (zum Inkubieren mind. einer Kartusche)
- 10: siebte Vorrichtung (zum Messen mind. eines Probenansatzes)
- 11: Testansatz
- 12: erstes Kompartiment (der Kartusche mit mind. einem Reagenz)
- 13: Reagenz
- 14: zweites Kompartiment (der Kartusche mit mind. einer Kontrolle)
- 15: Kontrolle
- 16: drittes Kompartiment (der Kartusche umfassend eine Messküvette)
- 17: Messküvette
- T3: dritte Temperatur
- T4: vierte Temperatur
- 18: Schacht
- 19: achte Vorrichtung (zur Steuerung der T1, T2, T3, T4)
- 20: neunte Vorrichtung (zur automatischen Verbringung Kartusche von 5 nach 2)
- 21: zehnte Vorrichtung (zur automatischen Verbringung Kartusche von 2 nach 6)
- 22: elfte Vorrichtung (zur automatischen Verbringung Kartusche von 6 nach 5)
- 23: Probe
- 24: Kalibrationskurve
- 25: Zentrallager
- 26: Herstellung Reagenzien
- 27: Herstellung Kartusche
- 28: Befüllung
- 30: Verfahren
- 31: Küvette 1
- 32: Küvette 2
- 33: Küvette 3
- 34: Küvette 4
- 35: Küvette 5
- 36: Küvette 6
- 37: Küvette 7
- 38: Küvette 8
- 39: Küvette 9
- 40: Küvette 10
- 41: Küvette 11
- 42: Küvette 12
- 43: Küvette 13
- 44: Küvette 14
- 45: Küvette 15
- 46: Küvette 16
- 47: Küvette 17
- 48: Küvette 18
- 49: Küvette 19
- 50: Küvette 20
- 51: Küvette 21
- 52: Küvette 22

## Patentansprüche

1. Automatisches Analysesystem (1) zum Analysieren einer Probe (23), das Analysesystem (1) umfassend:
- eine erste Vorrichtung (2) zur Lagerung von Kartuschen (3) bei einer ersten Temperatur (T1), wobei die erste Temperatur (T1) weniger als 268,15 Kelvin beträgt,
- eine dritte Vorrichtung (6) zur Lagerung von Kartuschen (3) bei einer zweiten Temperatur (T2), wobei die zweite Temperatur (T2) mehr als 273,15 Kelvin beträgt,
- eine vierte Vorrichtung (7) zum Erwärmen der Kartuschen (3) von der ersten Temperatur (T1) auf die zweite Temperatur (T2),
- eine fünfte Vorrichtung (8) zum Pipettieren mindestens einer Flüssigkeit,
- eine sechste Vorrichtung (9) zum Inkubieren mindestens einer Kartusche (3),
- eine siebte Vorrichtung (10) zum Messen mindestens eines Probenansatzes (11) der Probe (23), und
- Kartuschen (3), wobei die Kartuschen (3) jeweils mindestens ein erstes Kompartiment (12) mit mindestens einem Reagenz (13), mindestens ein zweites Kompartiment (14) mit mindestens einer Kontrolle (15), und mindestens ein drittes Kompartiment (16) umfassen, wobei das dritte Kompartiment (13) eine Messküvette (17) umfasst, wobei eine Messung des Testansatzes (11) mittels der siebten Vorrichtung (10) in der Messküvette (17) erfolgen kann,
wobei dem Analysesystem (1) mindestens eine Kartusche (3) zugeführt werden kann, wobei diese Kartusche eine dritte Temperatur (T3) aufweist, wobei die dritte Temperatur (T3) weniger als 268,15 Kelvin beträgt, wobei das Analysesystem (1) bevorzugt eine zweite Vorrichtung (4) zur Aufnahme einer Transportbox (5) umfasst, wobei bevorzugt die Transportbox (5) zum Transport mindestens einer Kartusche (3) bei der dritten Temperatur (T3) ausgestaltet ist.

2. Automatisches Analysesystem (1) nach Anspruch 1, wobei die Transportbox (5) weiter zum Transport mindestens einer Kartusche (3) bei einer vierten Temperatur (T4) ausgestaltet ist, wobei die vierte Temperatur (T4) 233,15 bis 323,15 Kelvin, bevorzugt 288,15 bis 298,15 Kelvin, besonders bevorzugt Raumtemperatur beträgt.

3. Automatisches Analysesystem (1) nach einem der vorhergehenden Ansprüche, wobei die dritte Vorrichtung (6) die vierte Vorrichtung (7), die fünfte Vorrichtung (8), die sechste Vorrichtung (9) und/oder die siebte Vorrichtung (10) wenigstens teilweise, bevorzugt vollständig umfasst.

4. Automatisches Analysesystem (1) nach einem der vorhergehenden Ansprüche, wobei die zweite Temperatur (T2) eine vorbestimmte, konstante Temperatur zwischen 293,15 und 315,15 Kelvin, bevorzugt zwischen 308,15 und 312,15 Kelvin ist, wobei besonders bevorzugt die zweite Temperatur (T2) 310,15 Kelvin beträgt.

5. Automatisches Analysesystem (1) nach einem der vorhergehenden Ansprüche, wobei die erste Temperatur (T1) weniger als 268,15 Kelvin, bevorzugt weniger als 263,15 Kelvin, besonders bevorzugt 255,15 bis 245,15 Kelvin beträgt.

6. Automatisches Analysesystem (1) nach einem der vorhergehenden Ansprüche, wobei die Flüssigkeit eine Probenflüssigkeit umfasst.

7. Automatisches Analysesystem (1) nach einem der vorhergehenden Ansprüche, wobei die fünfte Vorrichtung (8) eine Pipettiernadel zum Pipettieren von Proben, Reagenzien und/oder eines Verdünnungsmediums umfasst, wobei das Verdünnungsmedium bevorzugt eine Pufferlösung umfasst.

8. Automatisches Analysesystem (1) nach einem der vorhergehenden Ansprüche, wobei die zweite Vorrichtung (4) einen Schacht (18) umfasst, wobei der Schacht (18) zur Aufnahme mindestens einer Transportbox (5) ausgestaltet ist.

9. Automatisches Analysesystem (1) nach einem der vorhergehenden Ansprüche, wobei das Analysesystem eine achte Vorrichtung (19) zur Steuerung der ersten, zweiten, dritten und/oder vierten Temperatur (T1, T2, T3, T4) umfasst.

10. Automatisches Analysesystem (1) nach einem der vorhergehenden Ansprüche, wobei das Analysesystem (1) eine neunte Vorrichtung (20) zur automatischen Verbringung mindestens einer Kartusche (3) aus einer sich in der zweiten Vorrichtung (4) befindenden Transportbox (5) heraus und in die erste Vorrichtung (2) hinein umfasst.

11. Automatisches Analysesystem (1) nach einem der vorhergehenden Ansprüche, wobei das Analysesystem (1) eine zehnte Vorrichtung (21) zur automatischen Verbringung mindestens einer Kartusche (3) aus der ersten Vorrichtung (2) heraus und in die dritte Vorrichtung (6) hinein umfasst.

12. Automatisches Analysesystem (1) nach einem der vorhergehenden Ansprüche, wobei das Analysesystem (1) eine elfte Vorrichtung (17) zur automatischen Verbringung mindestens einer Kartusche (3) aus der dritten Vorrichtung (6) heraus in eine sich in der zweiten Vorrichtung (4) befindenden Transportbox (5) hinein umfasst.

13. Verfahren (30) zum Analysieren einer Probe (23) mittels eines automatischen Analysesystems (1) nach einem der Ansprüche 1 bis 12 unter Verwendung einer Kartusche (5) und bevorzugt einer Transportbox (5), das Verfahren umfassend die folgenden Schritte:
- Zuführung der Kartusche (3), bevorzugt in der Transportbox (5), zu dem automatischen Analysesystem (1), wobei die Kartusche (3) die erste Temperatur (T1) aufweist,
- bevorzugt Verbringung der Transportbox (5) mit der Kartusche (3) in die zweite Vorrichtung (4),
- bevorzugt Entnahme der Kartusche (3) aus der Transportbox (5) heraus und Zuführung in die erste Vorrichtung (2) mittels der neunten Vorrichtung (20), oder Zuführung der Kartusche (5) in die erste Vorrichtung (2),
- Entnahme der Kartusche (3) aus der ersten Vorrichtung (2) und Zuführung in die dritte Vorrichtung (6) hinein mittels der zehnten Vorrichtung (21),
- Erwärmung der Kartusche (3) von der ersten Temperatur (T1) auf die zweite Temperatur (T2) mittels der vierten Vorrichtung (7),
- Pipettierung mindestens einer Probe (23), mindestens eines Reagenzes (13) und mindestens einer Kontrolle (15) mittels der fünften Vorrichtung (8) umfassend die Herstellung eines Testansatzes (11),
- bevorzugt Inkubation des Testansatzes (11) in der Kartusche (3) mittels der sechsten Vorrichtung (9)
- Messung des Testansatzes (11) mittels der siebten Vorrichtung (10) in der Messküvette (17) des dritten Kompartiments (16) der Kartusche (3),
- Auswertung der Messung des Testansatzes (11) unter Verwendung mindestens einer Kalibrationskurve (24), die bevorzugt elektronisch an das Analysesystem (1) übermittelt wurde,
- bevorzugt Verbringung der Kartusche (3) aus der dritten Vorrichtung (6) hinaus in die Transportbox (5) hinein mittels der elften Vorrichtung (22),
- bevorzugt Transport der Kartusche (3) in der Transportbox (5) zu einer Entsorgungsstation, wobei die Kartusche (3) die vierte Temperatur (T4) aufweist.

14. Verfahren zum Analysieren einer Probe (23) nach Anspruch 13, wobei das Verfahren die Durchführung eines Faktor VIII-Bindungstests umfasst.

## Claims

1. Automatic analysis system (1) for analysing a sample (23), the analysis system (1) comprising:
- a first device (2) for storing cartridges (3) at a first temperature (T1), the first temperature (T1) being less than 268.15 kelvin,
- a third device (6) for storing cartridges (3) at a second temperature (T2), the second temperature (T2) being more than 273.15 kelvin,
- a fourth device (7) for heating the cartridges (3) from the first temperature (T1) to the second temperature (T2),
- a fifth device (8) for pipetting at least one liquid,
- a sixth device (9) for incubating at least one cartridge (3),
- a seventh device (10) for measuring at least one sample preparation (11) of the sample (23), and
- cartridges (3), wherein the cartridges (3) each comprise at least one first compartment (12) with at least one reagent (13), at least one second compartment (14) with at least one control (15), and at least one third compartment (16), the third compartment (13) comprising a measurement cuvette (17), wherein a measurement of the test preparation (11) can take place by means of the seventh device (10) in the measurement cuvette (17), wherein at least one cartridge (3) can be delivered to the analysis system (1), this cartridge having a third temperature (T3), the third temperature (T3) being less than 268.15 kelvin, the analysis system (1) preferably comprising a second device (4) for receiving a transport box (5), wherein the transport box (5) is preferably configured to transport at least one cartridge (3) at the third temperature (T3).

2. Automatic analysis system (1) according to Claim 1, wherein the transport box (5) is further configured to transport at least one cartridge (3) at a fourth temperature (T4), the fourth temperature (T4) being 233.15 to 323.15 kelvin, preferably 288.15 to 298.15 kelvin, particularly preferably room temperature.

3. Automatic analysis system (1) according to either of the preceding claims, wherein the third device (6) at least partially, preferably fully, comprises the fourth device (7), the fifth device (8), the sixth device (9) and/or the seventh device (10).

4. Automatic analysis system (1) according to one of the preceding claims, wherein the second temperature (T2) is a predetermined, constant temperature between 293.15 and 315.15 kelvin, preferably between 308.15 and 312.15 kelvin, the second temperature (T2) particularly preferably being 310.15 kelvin.

5. Automatic analysis system (1) according to one of the preceding claims, wherein the first temperature (T1) is less than 268.15 kelvin, preferably less than 263.15 kelvin, particularly preferably 255.15 to 245.15 kelvin.

6. Automatic analysis system (1) according to one of the preceding claims, wherein the liquid comprises a sample liquid.

7. Automatic analysis system (1) according to one of the preceding claims, wherein the fifth device (8) comprises a pipetting needle for pipetting samples, reagents and/or a dilution medium, the dilution medium preferably comprising a buffer solution.

8. Automatic analysis system (1) according to one of the preceding claims, wherein the second device (4) comprises a recess (18), the recess (18) being configured to receive at least one transport box (5).

9. Automatic analysis system (1) according to one of the preceding claims, wherein the analysis system comprises an eighth device (19) for controlling the first, second, third and/or fourth temperature (T1, T2, T3, T4).

10. Automatic analysis system (1) according to one of the preceding claims, wherein the analysis system (1) comprises a ninth device (20) for automatically bringing at least one cartridge (3) out of a transport box (5) contained in the second device (4) and into the first device (2).

11. Automatic analysis system (1) according to one of the preceding claims, wherein the analysis system (1) comprises a tenth device (21) for automatically bringing at least one cartridge (3) out of the first device (2) and into the third device (6).

12. Automatic analysis system (1) according to one of the preceding claims, wherein the analysis system (1) comprises an eleventh device (17) for automatically bringing at least one cartridge (3) out of the third device (6) into a transport box (5) contained in the second device (4).

13. Method (30) for analysing a sample (23) by means of an automatic analysis system (1) according to one of Claims 1 to 12 by using a cartridge (5) and preferably a transport box (5), the method comprising the following steps:
- delivering the cartridge (3), preferably in the transport box (5), to the automatic analysis system (1), the cartridge (3) having the first temperature (T1),
- preferably bringing the transport box (5) with the cartridge (3) into the second device (4),
- preferably removing the cartridge (3) from the transport box (5) and delivering it into the first device (2) by means of the ninth device (20), or delivering the cartridge (5) into the first device (2),
- removing the cartridge (3) from the first device (2) and delivering it into the third device (6) by means of the tenth device (21),
- heating the cartridge (3) from the first temperature (T1) to the second temperature (T2) by means of the fourth device (7),
- pipetting at least one sample (23), at least one reagent (13) and at least one control (15) by means of the fifth device (8) comprising the production of a test preparation (11),
- preferably incubating the test preparation (11) in the cartridge (3) by means of the sixth device (9),
- measuring the test preparation (11) by means of the seventh device (10) in the measurement cuvette (17) of the third compartment (16) of the cartridge (3),
- evaluating the measurement of the test preparation (11) by using at least one calibration curve (24), which has preferably been transmitted electronically to the analysis system (1),
- preferably bringing the cartridge (3) out of the third device (6) into the transport box (5) by means of the eleventh device (22),
- preferably transporting the cartridge (3) in the transport box (5) to a disposal station, the cartridge (3) having the fourth temperature (T4).

14. Method for analysing a sample (23) according to Claim 13, wherein the method comprises carrying out a factor VIII binding test.

## Revendications

1. Système (1) automatique d'analyse pour l'analyse d'un échantillon (23), le système (1) d'analyse comprenant :
- un premier dispositif (2) pour le stockage de cartouches (3) à une première température (T1), la première température (T1) étant inférieure à 268,15 Kelvin,
- un troisième dispositif (6) pour le stockage de cartouches (3) à une deuxième température (T2), la deuxième température (T2) étant supérieure à 273,15 Kelvin,
- un quatrième dispositif (7) pour le chauffage des cartouches (3) de la première température (T1) à la deuxième température (T2),
- un cinquième dispositif (8) pour le pipetage d'au moins un liquide,
- un sixième dispositif (9) pour l'incubation d'au moins une cartouche (3),
- un septième dispositif (10) pour la mesure d'au moins une préparation (11) de l'échantillon (23), et
- des cartouches (3), dans lequel les cartouches (3) comprennent chacune au moins un premier compartiment (12) ayant au moins un réactif (13), au moins un deuxième compartiment (14) ayant au moins un témoin (15) et au moins un troisième compartiment (16), dans lequel le troisième compartiment (13) comprend une cuvette (17) de mesure, dans lequel une mesure de la préparation (11) de test peut s'effectuer dans la cuvette (17) de mesure au moyen du septième dispositif (10),
dans lequel au moins une cartouche (3) peut être envoyée au système (1) d'analyse, dans lequel cette cartouche a une troisième température (T3), dans lequel la troisième température (T3) est inférieure à 268,15 Kelvin, dans lequel le système (1) d'analyse comprend de préférence un deuxième dispositif (4) de réception d'une boîte (5) de transport, dans lequel de préférence la boîte (5) de transport est conformée pour le transport d'au moins une cartouche (3) à la troisième température (T3).

2. Système (1) automatique d'analyse suivant la revendication 1, dans lequel la boîte (5) de transport est conformée en outre pour le transport d'au moins une cartouche (3) à une quatrième température (T4), la quatrième température (T4) allant de 233,15 à 323,15 Kelvin, de préférence de 288,15 à 298,15 Kelvin, en étant d'une manière particulièrement préférée la température ambiante.

3. Système (1) automatique d'analyse suivant l'une des revendications précédentes, dans lequel le troisième dispositif (6) entoure au moins en partie, de préférence complètement, le quatrième dispositif (7), le cinquième dispositif (8), le sixième dispositif (9) et/ou le septième dispositif (10).

4. Système (1) automatique d'analyse suivant l'une des revendications précédentes, dans lequel la deuxième température (T2) est une température constante déterminée à l'avance comprise entre 293,15 et 315,15 Kelvin, de préférence entre 308,15 et 312,15 Kelvin, la deuxième température (T2) étant d'une manière particulièrement préférée de 310,15 Kelvin.

5. Système (1) automatique d'analyse suivant l'une des revendications précédentes, dans lequel la première température (T1) est inférieure à 268,15 Kelvin, de préférence inférieure à 263,15 Kelvin, en allant, d'une manière particulièrement préférée, de 255,15 à 245,15 Kelvin.

6. Système (1) automatique d'analyse suivant l'une des revendications précédentes, dans lequel le liquide comprend un liquide d'échantillon.

7. Système (1) automatique d'analyse suivant l'une des revendications précédentes, dans lequel le cinquième dispositif (8) comprend une aiguille de pipetage pour le pipetage d'échantillons, de réactifs et/ou d'un milieu de dilution, dans lequel le milieu de dilution comprend de préférence une solution tampon.

8. Système (1) automatique d'analyse suivant l'une des revendications précédentes, dans lequel le deuxième dispositif (4) comprend un puits (18), le puits (18) étant conformé pour la réception d'au moins une boîte (5) de transport.

9. Système (1) automatique d'analyse suivant l'une des revendications précédentes, dans lequel le système d'analyse comprend un huitième dispositif (19) pour se rendre maître de la première, deuxième, troisième et/ou quatrième températures (T1, T2, T3, T4).

10. Système (1) automatique d'analyse suivant l'une des revendications précédentes, dans lequel le système (1) d'analyse comprend un neuvième dispositif (20) pour sortir automatiquement au moins une cartouche (3) d'une boîte (5) de transport se trouvant dans le deuxième dispositif (4) et la mettre dans le premier dispositif (2).

11. Système (1) automatique d'analyse suivant l'une des revendications précédentes, dans lequel le système (1) d'analyse comprend un dixième dispositif (21) pour sortir automatiquement au moins une cartouche (3) du premier dispositif (2) et la mettre dans le troisième dispositif (6).

12. Système (1) automatique d'analyse suivant l'une des revendications précédentes, dans lequel le système (1) d'analyse comprend un onzième dispositif (17) pour sortir automatiquement au moins une cartouche (3) du troisième dispositif (6) et la mettre dans une boîte (5) de transport se trouvant dans le deuxième dispositif (4).

13. Procédé (30) d'analyse d'un échantillon (23), au moyen d'un système (1) automatique d'analyse suivant l'une des revendications 1 à 12, en utilisant une cartouche (5) et de préférence une boîte (5) de transport, le procédé comprenant les stades suivants :
- envoi de la cartouche (3), de préférence dans la boîte (5) de transport, au système (1) automatique d'analyse de la cartouche (3) ayant la première température (T1),
- mise de préférence de la boîte (5) de transport ayant la cartouche (3) dans le deuxième dispositif (4),
- de préférence sortie de la cartouche (3) de la boîte (5) de transport et envoi de celle-là au premier dispositif (2) au moyen du neuvième dispositif (20) de transport ou envoi de la cartouche (5) au premier dispositif (2),
- sortie de la cartouche (3) du premier dispositif (2) et envoi de celle-ci dans le troisième dispositif (6) au moyen du dixième dispositif (21) de transport,
- chauffage de la cartouche (3) de la première température (T1) à la deuxième température (T2) au moyen du quatrième dispositif (7),
- pipetage d'au moins un échantillon (23), d'au moins un réactif (13) et d'au moins un témoin (15) au moyen du cinquième dispositif (8) comprenant la préparation d'une préparation (11) de test,
- de préférence incubation de la préparation (11) de test dans la cartouche (3) au moyen du sixième dispositif (9),
- mesure de la préparation (11) de test au moyen du septième dispositif (10) dans la cuvette (17) de mesure du troisième compartiment (16) de la cartouche (3),
- évaluation de la mesure de la préparation (11) de test en utilisant au moins une courbe (24) d'étalonnage, qui a été transmise, de préférence électroniquement, au système (1) d'analyse,
- de préférence sortie de la cartouche (3) du troisième dispositif (6) pour la mettre dans la boîte (5) de transport au moyen du onzième dispositif (22),
- de préférence transport de la cartouche (3) dans la boîte (5) de transport à un poste d'évacuation, la cartouche (3) ayant la quatrième température (T4).

14. Procédé d'analyse d'un échantillon (23) suivant la revendication 13, dans lequel le procédé comprend l'exécution d'un test de fixation du facteur VIII.
